# EUROPÄISCHE PATENTANMELDUNG

(11) **EP 1 078 648 A1**
(43) Veröffentlichungstag der Anmeldung: **28.02.2001**
(21) Anmeldenummer: 00116462.3
(22) Anmeldetag: 29.07.2000
(51) Int. Cl.: A61N 1/36, A61N 1/18, H02G 3/08, H01R 9/24

(54) **Anschlusskörper zum Anschliessen einer Vielzahl von elektrisch leitfähigen Radiofrequenznadeln an eine elektrische Spannungsquelle**

(30) Priorität: 26.08.1999 DE 29914848 U
(71) Anmelder: Meyer, Frank- Egbert, Dr., 53340 Meckenheim (DE)
(72) Erfinder: Meyer, Frank- Egbert, Dr., 53340 Meckenheim (DE)
(74) Vertreter: Müller-Gerbes, Margot, Dipl.-Ing.

(57) **Zusammenfassung**

Die Erfindung betrifft einen Anschlußkörper zum Anschließen einer Vielzahl von elektrisch leitfähigen Radiofrequenznadeln an eine elektrische Spannungsquelle zur Nervenstimulation und/oder Thermokoagulation eines Patienten, umfassend einen Korpus mit mindestens einem Eingang, an den die elektrische Spannungsquelle anschließbar ist und eine Vielzahl von mit dem mindestens einem Eingang elektrisch verbindbaren Ausgängen, an die jeweils eine Radiofrequenznadel anschließbar ist.

## Beschreibung

Die Erfindung betrifft einen Anschlußkörper zum Anschließen einer Vielzahl von elektrisch leitfähigen Radiofrequenznadeln an eine elektrische Spannungsquelle zur Nervenstimulation und/oder Thermokoagulation eines Patienten.

Die Stimulation von Nerven mittels elektrisch leitfähiger sog. Radiofrequenznadeln, die im Bereich der Nervenumgebung in einen Patienten eingestochen und an eine elektrische Spannungsquelle angeschlossen werden, stellt eine immer häufiger zum Einsatz kommende medizinische Anwendung dar. Insbesondere zur Behandlung von Schmerzpatienten stellt überdies die Bewirkung einer sog. Facettenblockade eine immer größere Bedeutung erlangende Behandlung dar, bei der die für das Schmerzempfinden des Patienten verantwortlichen Nerven, insbesondere im Bereich der Wirbelsäule, blockiert werden.

Hierzu ist es erforderlich, eine größere Anzahl von sog. Radiofrequenznadeln in genau definierten Einstichpunkten, z. B. nahe der Wirbelsäule einzustechen, beispielsweise bis zu 14 solcher Radiofrequenznadeln, und diese aufeinanderfolgend mit einer elektrischen Spannungsquelle zusammen mit einer Gegenelektrode auf der Haut des Patienten zu verbinden, wodurch in unmittelbarer Umgebung der Spitze einer eingestochenen Radiofrequenznadel eine Erwärmung des den Nerv umgebenden Gewebes auf bis zu etwa 80°C bewirkt wird, was eine gewünschte punktuelle Blockierung des Nervs hervorruft.

Die bisherige Vorgehensweise beim Erzeugen einer solchen Facettenblockade ist es, zunächst die benötigte Anzahl von Radiofrequenznadeln unter Räntgendurchleuchtung in die zuvor festgelegten Positionen im Patienten einzustechen und nachfolgend jeweils manuell für eine gewisse für die Erwärmung auf etwa 80°C ausreichende Zeitdauer mit der elektrischen Spannungsquelle zu verbinden.

Da jede Radiofrequenznadel für den Anschluß an die elektrische Spannungsquelle ein elektrisches Anschlußkabel aufweist und meist noch zusätzlich über einen Zuspritzschlauch für das Zuspritzen eines Lokalanästhetikums in den Einstichbereich verfügt, führt dies bisher je nach Anzahl der erforderlichen eingestochenen Radiofrequenznadeln zu einer großen Anzahl von mehr oder weniger lose herumliegenden elektrischen Anschlußkabeln und Zuspritzschläuchen für die einzelnen Radiofrequenznadeln, was eine unerwünschte Handhabungserschwernis mit sich bringt und zudem große Sorgfalt und Aufmerksamkeit des behandelnden Arztes erfordert.

Darüber hinaus sind bisher sowohl die Radiofrequenznadeln am freien Ende ihrer elektrischen Anschlußleitung wie auch eine Zuführleitung von der elektrischen Spannungsquelle an ihren miteinander zu verbindenden Enden mit sog. Krokodilklemmen ausgerüstet, die sich zwar zur Herstellung einer elektrischen Verbindung einfach aneinanderfügen lassen, jedoch den wesentlichen Nachteil aufweisen, daß eine jede solche über das Aneinanderstecken von Krokodilklemmen bewirkte elektrische Verbindung stets unterschiedliche Leitungsquerschnitte aufweist, wodurch sich der elektrische Widerstand im Bereich der Krokodilklemmen bei jedem Verbindungsaufbau ändert. Zur Erzeugung der gewünschten Erwärmung des Gewebes des Patienten auf ca. 80°C ist jedoch eine exakt definierte Leistung notwendig, die aufgrund der wechselnden elektrischen Widerstände innerhalb der Verbindung Schwankungen unterliegt, so daß das Ergebnis verbesserungswürdig erscheint.

Der Erfindung liegt von daher die Aufgabe zugrunde, den Anschluß einer Vielzahl von elektrisch leitfähigen Radiofrequenznadeln an eine elektrische Spannungsquelle übersichtlicher und einfacher zu gestalten und darüber hinaus stets gleiche Widerstandswerte für jede an die elektrische Spannungsquelle anzuschließende Radiofrequenznadel sicherzustellen.

Zur Lösung dieser gestellten Aufgabe wird ein Anschlußkörper gemäß den im Patentanspruch 1 angegebenen Merkmalen vorgeschlagen.

Vorteilhafte Ausgestaltungen und Weiterbildungen des erfindungsgemäßen Anschlußkörpers sind Gegenstand der Unteransprüche.

Zur Lösung der gestellten Aufgabe schlägt die Erfindung einen Anschlußkörper zum Anschließen einer Vielzahl von elektrisch leitfähigen Radiofrequenznadeln an eine elektrische Spannungsquelle zur Nervenstimulation und/oder zum Bewirken einer Facettenblockade eines Patienten vor, die einen Korpus mit mindestens einem Eingang umfaßt, an den die elektrische Spannungsquelle anschließbar ist und ferner eine Vielzahl von mit dem mindestens einem Eingang elektrisch verbindbaren Ausgängen umfaßt, an die jeweils eine Radiofrequenznadel anschließbar ist.

Auf diese Weise wird es möglich, nach dem Einstechen der Radiofrequenznadeln in die zuvor ausgewählten Einstichpunkte des Patienten sämtliche Radiofrequenznadeln mit ihren jeweils zugehörigen elektrischen Anschlußkabeln an je einen Ausgang des erfindungsgemäß vorgeschlagenen Anschlußkörpers anzuschließen, wodurch keine frei herumliegenden und nur mit großer Aufmerksamkeit auswählbare Leitungsenden von den Radiofrequenznadeln mehr vorliegen. Sodann braucht die elektrische Spannungsquelle lediglich noch an den mindestens einen Eingang des erfindungsgemäß vorgeschlagenen Anschlußkörpers angeschlossen zu werden und nacheinander der mit der elektrischen Spannungsquelle verbundene Eingang mit je einem Ausgang, an dem jeweils eine Radiofrequenznadel angeschlossen ist, verbunden zu werden, wodurch die Handhabung auch bei einer sehr großen Anzahl von Radiofrequenznadeln, wie es z. B. für die Bewirkung von Facettenblockaden notwendig ist, extrem vereinfacht wird.

Ferner ergibt sich durch die Ausbildung eines erfindungsgemäßen Anschlußkörpers mit einer Vielzahl von Ausgängen, an die die Radiofrequenznadeln angeschlossen werden, der große Vorteil, daß nach dem Anschließen an den elektrischen Anschlußkörper an den Radiofrequenznadeln bzw. deren an die Ausgänge des Anschlußkörpers angeschlossenen elektrischen Anschlußleitungen keinerlei Manipulationen mehr notwendig sind, was die Gefahr von nachträglichen Veränderungen der Einstichposition der Radiofrequenznadeln durch solche Manipulationen ausschaltet.

Darüber hinaus werden durch den Anschluß aller Radiofrequenznadeln an jeweils einen Ausgang des erfindungsgemäß vorgeschlagenen Anschlußkörpers und die Ausbildung von mindestens einem Eingagn für den Anschluß der elektrischen Spannungsquelle stets gleiche Widerstände für die elektrische Verbindung geschaffen, so daß an allen Radiofrequenznadeln die gleiche Leistung der elektrischen Spannungsquelle anliegen kann.

Für die Ausgestaltung des erfindungsgemäß vorgeschlagenen Anschlußkörpers sind im Rahmen der Erfindung mehrere Möglichkeiten vorgesehen.

Ein besonders einfacher Aufbau des erfindungsgemäßen Anschlußkörpers wird dadurch erreicht, daß der Korpus eine Vielzahl von Durchgangsbohrungen aufweist, in die jeweils ein elektrisch leitfähiger Kontaktstift eingesetzt ist, so daß an einem Ende jedes Kontaktstiftes ein Eingang für den Anschluß der elektrischen Spannungsquelle und am gegenüberliegenden Ende ein Ausgang für den Anschluß einer Radiofrequenznadel ausbildbar ist. Bei dieser Ausführungsform des erfindungsgemäßen Anschlußkörpers liegen demgemäß Eingänge und Ausgänge in gleicher Anzahl vor. Die Radiofrequenznadeln werden jeweils an einen Ausgang des erfindungsgemäßen Anschlußkörpers angeschlossen und die elektrische Spannungsquelle wird der Reihe nach mit den verschiedenen in gleicher Anzahl vorliegenden Eingängen des erfindungsgemäßen Anschlußkörpers verbunden, wodurch jeweils eine Radiofrequenznadel mit der elektrischen Spannungsquelle elektrisch verbunden wird und die gewünschte Erwärmung des Gewebes zur Ausbildung der Facettenblockade bewirkt.

Vorteilhaft sind hierbei die Kontaktstifte mit einer solchen Länge ausgebildet, da sie mit ihren beiden Enden aus dem Korpus hervorragen. Hierdurch sind die Kontaktstifte in ihrem aus dem erfindungsgemäßen Anschlußkörper herausragenden Bereich frei zugänglich und können beispielsweise durch entsprechende an den Anschlußleitungen der Radiofrequenznadeln und/oder der Zuführleitung von der elektrischen Spannungsquelle ausgebildeten Steckbuchsen oder Krokodilklemmen kontaktiert werden, um eine elektrische Verbindung zwischen der elektrischen Spannungsquelle am Eingang und einer am entsprechenden Ausgang angeschlossenen Radiofrequenznadel herzustellen.

Eine weitere vorteilhafte Ausführungsform des erfindungsgemäßen Anschlußkörpers sieht vor, daß eine einen Eingang ausbildende Eingangsbuchse für den Anschluß der elektrischen Spannungsquelle und eine Vielzahl von Ausgängen ausbildende Ausgangsbuchsen für den Anschluß jeweils einer Radiofrequenznadel vorgesehen sind und der Anschlußkörper einen Schalter aufweist, mittels dessen je eine Ausgangsbuchse mit der Eingangsbuchse des erfindungsgemäßen Anschlußkörpers elektrisch verbindbar ist.

Bei dieser Ausführungsform des erfindungsgemäßen Anschlußkörpers ist es nicht nur möglich, sämtliche Radiofrequenznadeln nach ihrem Einstechen in den Patienten mit ihren entsprechenden elektrischen Anschlußleitungen ausgangsseitig an den erfindungsgemäßen Anschlußkörper anzuschließen, sondern auch die elektrische Spannungsquelle wird eingangsseitig mit ihrer entsprechenden Zuführleitung bereits an die Eingangsbuchse des erfindungsgemäßen Anschlußkörpers angeschlossen, so daß weitere Manipulationen nicht mehr erforderlich sind. Nunmehr wird lediglich durch Betätigung des auf dem Anschlußkörper ausgebildeten Schalters jeweils eine elektrische Verbindung zwischen einer an einen Ausgang angeschlossenen Radiofrequenznadel und der an den einen Eingang angeschlossenen elektrischen Spannungsquelle hergestellt, um aufeinanderfolgend sämtliche an den erfindungsgemäßen Anschlußkörper angeschlossenen Radiofrequenznadeln mit der elektrischen Spannung zu beaufschlagen und die gewünschte Facettenblockade zu bewirken.

Um eine besonders einfache Handhabung zu erzielen, wird vorgeschlagen, daß der Schalter der Anzahl Ausgangsbuchsen entsprechende Schaltstellungen für die elektrische Verbindung je einer Ausgangsbuchse mit der Eingangsbuchse und eine weitere Schaltstellung aufweist, in welcher keine der Ausgangsbuchsen mit der Eingangsbuchse verbunden ist, d. h. eine Nullstellung, bei der der elektrische Anschluß bewirkt werden kann und nach Abschluß der Facettenblockade alle angeschlossenen Radiofrequenznadeln spannungslos sind.

Eine noch größere Handhabungssicherheit kann dadurch erreicht werden, daß der Schalter der Anzahl Ausgangsbuchsen entsprechende Schaltstellungen für die elektrische Verbindung je einer Ausgangsbuchse mit der Eingangsbuchse aufweist und zwischen zwei benachbarten Schaltstellungen zum Verbinden einer Ausgangsbuchse mit der Eingangsbuchse jeweils eine weitere Schaltstellung vorgesehen ist, in welcher keine Ausgangsbuchse mit der Eingangsbuchse elektrisch verbunden ist. Hierdurch ist es möglich, durch aufeinanderfolgendes Aufsuchen aller Schaltstellungen des Schalters sämtliche an den erfindungsgemäßen Anschlußkörper angschlossenen Radiofrequenznadeln der Reihe nach mit der elektrischen Spannungsquelle zu verbinden, wobei zwischen jedem aufeinanderfolgenden Verbinden von benachbarten Radiofrequenznadeln eine Nullstellung eingefügt ist, in welcher keine der Radiofrequenznadeln an die elektrische Spannungsquelle angeschlossen ist. Hierdurch wird es dem behandelnden Arzt möglich, nach jeder Beaufschlagung einer Radiofrequenznadel mit der elektrischen Spannung der elektrischen Spannungsquelle eine Unterbrechung einzulegen, in welcher beispielsweise eine Überprüfung der soeben bewirkten Behandlung vorgenommen werden kann.

Der im Rahmen der Erfindung vorgeschlagene Schalter des erfindungsgemäßen Anschlußkörpers ist vorteilhaft als Drehschalter ausgebildet, der sich besonders gut für das aufeinanderfolgende Aufsuchen sämtlicher Schaltstellungen eignet.

Um eine möglichst universelle Anwendbarkeit des erfindungsgemäßen Anschlußkörpers an verschiedene Radiofrequenznadeln und deren Anschlußleitungen sowie elektrische Spannungsquellen, z. B. an sich bekannte Nervenstimulatoren zu gewährleisten, sind der mindestens eine Eingang und die Ausgänge des erfindungsgemäßen Anschlußkörpers für den Anschluß von Steckbuchsen und/oder Krokodilklemmen vorgesehen.

Vorteilhaft weist der Korpus des erfindungsgemäßen Anschlußkörpers eine etwa quaderförmige Gestalt auf, wobei der mindestens eine Eingang auf einer Längsseite und die Ausgänge auf der gegenüberliegenden Längsseite des Korpus ausgebildet sind, wodurch sich eine eindeutige räumliche Trennung von Eingängen und Ausgängen des Anschlußkörpers ergibt, was der Übersichtlichkeit des erfindungsgemäßen Anschlußkörpers förderlich ist.

Der Korpus kann beispielsweise aus einem thermoplastischen Kunststoff wie PVC hergestellt sein, beispielsweise aus einem massiven spritzgegossenen Block, der eine ausreichende elektrische Isolation zwischen den einzelnen Eingängen und Ausgängen sicherstellt.

Die Erfindung wird nachfolgend anhand von Ausführungsbeispielen in der Zeichnung näher erläutert. Es zeigen:
- Figur 1: eine Ausführungsform eines erfindungsgemäßen Anschlußkörpers in der Aufsicht,
- Figur 2: eine weitere Ausführungsform eines erfindungsgemäßen Anschlußkörpers in der Aufsicht,
- Figur 3: eine weitere Ausführungsform des erfindungsgemäßen Anschlußkörpers in der Aufsicht,
- Figur 4a: eine Aufsicht auf den Korpus des erfindungsgemäßen Anschlußkörpers gemäß Figur 1,
- Figur 4b: die Seitenansicht des Korpus gemäß Pfeil X in Figur 4a,
- Figur 5: in schematisierter Darstellung eine zum Zusammenwirken mit dem erfindungsgemäßen Anschlußkörper geeignete Radiofrequenznadel.

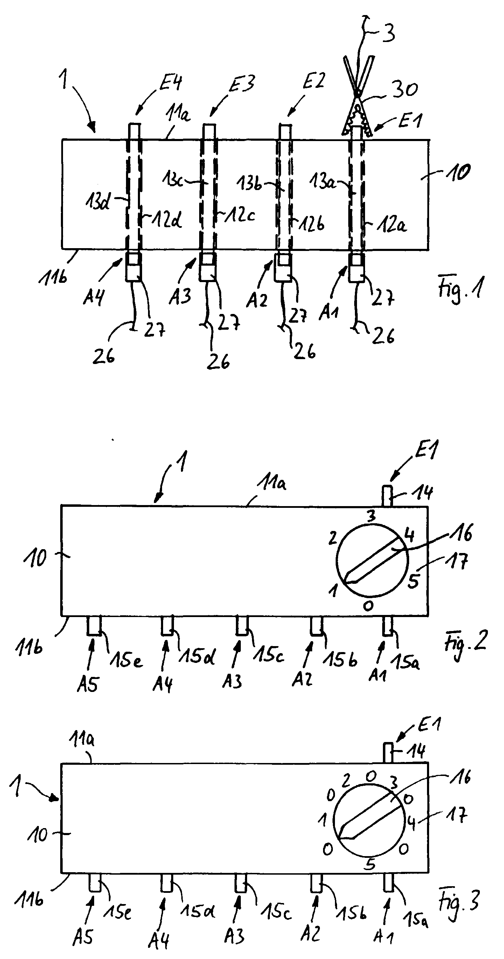

In der Figur 1 ist eine Ausführungsform eines elektrischen Anschlußkörpers 1 zum Anschließen einer Vielzahl von elektrisch leitfähigen Radiofrequenznadeln an eine elektrische Spannungsquelle zur Nervenstimulation und/oder Thermokoagulation bei der Schmerztherapie eines Patienten dargestellt.

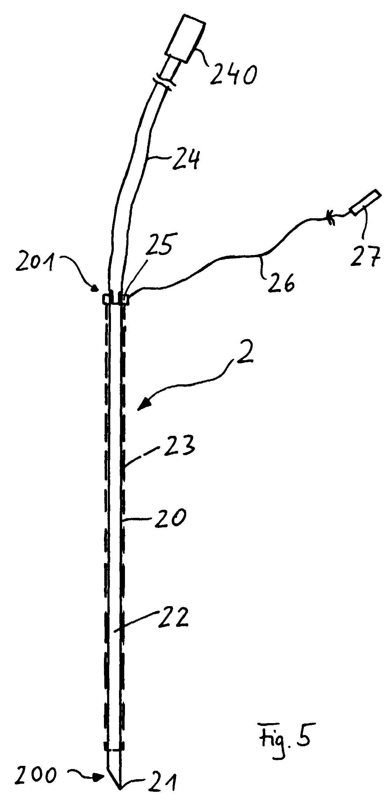

Unter Bezugnahme auf die Figur 5 wird zunächst erläuternd der Aufbau einer sog. Radiofrequenznadel 2 erläutert, die zum Zusammenwirken mit dem Anschlußkörper 1 vorgesehen ist.

Eine solche Radiofrequenznadel 2 weist einen elektrisch leitfähigen Nadelkörper 20, beispielsweise aus Metall auf, der ein durchgehendes Lumen 22 nach Art einer Kanüle umgibt. An seinem distalen Ende 200 weist der Nadelkörper 20 eine angeschliffene Spitze 21 auf, während am gegenüberliegenden proximalen Ende 201 der elektrisch leitfähige Nadelkörper 20 über ein elektrisches Anschlußelement 25 mit einer elektrischen Anschlußleitung 26 und einer am Ende derselben ausgebildeten Anschlußbuchse 27 elektrisch verbunden ist. Der Nadelkörper 20 der Radiofrequenznadel 2 ist mittels einer in der Figur 5 strichliert angedeuteten Isolierung, beispielsweise einem Überzug 23 aus einem geeigneten Kunststoffmaterial elektrisch isolierend überzogen, lediglich im Bereich des distalen Endes 200 ist der elektrisch leitfähige Nadelkörper 20 nicht mit dem Überzug 23 versehen und von daher im Bereich des distalen Endes 200 nicht gegenüber der Umgebung isoliert.

Ferner ist der Figur 5 entnehmbar, daß an das proximale Ende 201 des Nadelkörpers 20 anschließend ein Zuspritzschlauch 24 mit einem an dessen freien Ende ausgebildeten Anschlußelement 240 vorgesehen ist, an welches beispielsweise eine Spritze zum Zuführen eines Lokalanästhetikums über den Zuspritzschlauch 24 in das durchgehende Lumen 22 des Nadelkörpers 20 anschließbar ist.

Eine Vielzahl von solchen in der Figur 5 dargestellten Radiofrequenznadeln 2 wird beispielsweise zur Nervenstimulation und insbesondere zum Bewirken einer sog. Facettenblockade verwendet. Dazu werden derartige Radiofrequenznadeln 2 in ausreichender Anzahl, beispielsweise bis zu 14 solcher Radiofrequenznadeln 2 an vorher exakt festgelegten Einstichpunkten in den Körper eines Patienten eingestochen, beispielsweise beidseits der Wirbelsäule im Bereich eines schmerzerzeugenden Nervs. Wenn nun an die Anschlußbuchse 27 der Radiofrequenznadel 2 eine elektrische Spannungsquelle, beispielsweise ein an sich handelsüblicher Nervenstimulator angeschlossen wird, wird die Radiofrequenznadel 2 über die elektrische Anschlußleitung 26 und den elektrisch leitfähigen Nadelkörper unter eben diese elektrische Spannung gesetzt, die am unisolierten distalen Ende 200 abgegriffen werden kann. Unter Hinzunahme einer ebenfalls am Körper des Patienten befestigten Gegenelektrode kann sodann ein elektrischer Strom erzeugt werden, der im Bereich des distalen Endes 200 der Radiofrequenznadel 2 eine lokale Erwärmung des Gewebes des Patienten bis auf etwa 80°C bewirkt, wodurch eine Blockade des in unmittelbarer Nähe befindlichen Nervs in an sich bekannter Weise bewirkt wird.

Um nun die große Anzahl von für eine Facettenblockade benötigten Radiofrequenznadeln 2 in besonders einfacher und gleichmäßiger Weise mit der elektrischen Spannungsquelle aufeinanderfolgend verbinden zu können, wird der bereits eingangs erwähnte elektrische Anschlußkörper 1 gemäß Figur 1 verwendet.

Dieser elektrische Anschlußkörper 1 weist einen etwa quaderförmigen und massiven Korpus 10 aus einem Kunststoff, wie PVC auf.

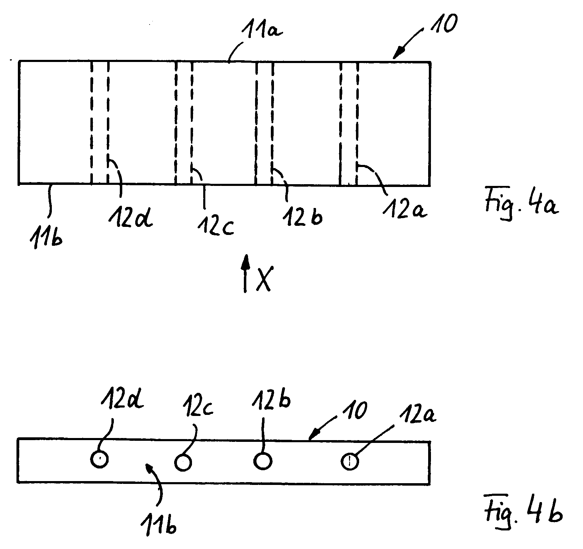

Wie auch in näheren Einzelheiten aus den Darstellungen gemäß Figur 4a und 4b ersichtlich, weist der Korpus 10 mehrere, hier 4 quer von einer Längsseite 11a bis zur anderen Längsseite 11b durch den Korpus 10 verlaufende Durchgangsbohrungen 12a, 12b, 12c, 12d auf, die in etwa gleichem Abstand und achsparallel zueinander angeordnet sind. In diese Durchgangsbohrungen 12a, 12b, 12c, 12d ist jeweils ein elektrisch leitfähiger Kontaktstift 13a, 13b, 13c, 13d eingesetzt, beispielsweise klemmend eingeschoben, wobei jeder Kontaktstift 13a, 13b, 13c, 13d eine solche Länge aufweist, daß er über beide Längsseiten 11a, 11b des Korpus 10 mit seinen jeweiligen Endbereichen hervorragt.

Die auf der Längsseite 11a des Korpus 10 hervorragenden Endbereiche der Kontaktstifte 12a, 12b, 12c, 12d bilden hierbei Eingänge E1, E2, E3, E4 aus, während die auf der Längsseite 11b des Korpus 10 hervorragenden Endbereiche aller Kontaktstifte 13a, 13b, 13c, 13d Ausgänge A1, A2, A3, A4 des Anschlußkörpers 1 ausbilden.

Der solchermaßen ausgebildete und in der Figur 1 dargestellte Anschlußkörper 1 weist von daher mehrere, hier 4 Eingänge E1, E2, E3, E4 und eine gleiche Anzahl Ausgänge A1, A2, A3, A4 auf, wobei jeweils ein Eingang E1 mit dem Ausgang A1 elektrisch verbunden ist, ebenso wie die übrigen Eingänge E2, E3, E4 mit den weiteren Ausgängen A2, A3, A4 jeweils separat elektrisch verbunden sind.

Mit einem solchermaßen ausgebildeten Anschlußkörper 1 ist es möglich, eine Vielzahl von vorangehend bereits erläuterten Radiofrequenznadeln 2 elektrisch mit einer elektrischen Spannungsquelle zu verbinden, um beispielsweise eine Facettenblockade zu bewirken. Hierzu werden die Radiofrequenznadeln 2 in die vorbestimmten Einstichpositionen im Körper des Patienten eingestochen und sodann sämtliche Radiofrequenznadeln 2 mit ihren in der Figur 1 angedeuteten elektrischen Anschlußleitungen 26 und den am freien Ende angeordneten Anschlußbuchsen 27 auf je einen Ausgang A1, A2, A3 oder A4 aufgesteckt.

Nach dieser solchermaßen bewirkten elektrischen Verbindung der Radiofrequenznadeln 2 mit dem Anschlußkörper 1 ist keine weitere Manipulation mehr an den Radiofrequenznadeln 2 bzw. deren elektrischen Anschlußleitungen 26 nötig, so daß der Gefahr einer nachträglichen unerwünschten Positionsveränderung der Radiofrequenznadeln 2 im Körper des Patienten vorgebeugt ist.

Zur Bewirkung der Facettenblockade wird nun die in der Figur 1 angedeutete Zuleitung 3 der elektrischen Spannungsquelle, beispielsweise des Nervenstimulators, die an ihrem freien Ende ein geeignetes Anschlußelement, beispielsweise die in der Figur 1 dargestellte Krokodilklemme 30 aufweist, aufeinanderfolgend mit je einem Ausgang A1, A2, A3 bzw. A4 und der jeweils hieran angeschlossenen Radiofrequenznadel 2 verbunden. Zu diesem Zweck wird die Krokodilklemme 30 am Ende der Zuleitung 3 von der elektrischen Spannungsquelle nacheinander auf die die Eingänge E1, E2, E3, E4 ausbildenden Enden der Kontaktstifte 13a, 13b, 13c, 13d aufgesteckt, wodurch jeweils eine elektrische Verbindung zwischen der elektrischen Spannungsquelle und einer an einen Ausgang angeschlossenen Radiofrequenznadel 2 bewirkt wird.

Auf diese Weise wird das aufeinanderfolgende Beaufschlagen aller für die Facettenblockade benötigten Radiofrequenznadeln 2 mit elektrischer Spannung der elektrischen Spannungsquelle erheblich vereinfacht. Gleichzeitig liegen bei jeder Verbindung der elektrischen Spannungsquelle über die Eingänge E1, E2, E3, E4 mit den an die Ausgänge A1, A2, A3, A4 angeschlossenen Radiofrequenznadeln 2 stets gleiche Leitungswiderstände durch konstante Anschlußquerschnitte vor, so daß an jeder Radiofrequenznadel 2 die gleiche Leistung der elektrischen Spannungsquelle abgegeben werden kann.

Eine weitere mögliche Ausführungsform des erfindungsgemäßen Anschlußkörpers 1 ist in der Figur 2 dargestellt.

Hierbei weist der Anschlußkörper 1 wiederum einen massiven quaderförmigen Korpus 10 aus einem geeigneten Kunststoff, wie PVC auf, verfügt jedoch auf der Längsseite 11a lediglich über eine Eingangsbuchse 14 zur Ausbildung eines einzigen Eingangs E1, an den die elektrische Spannungsquelle anschließbar ist. Auf der gegenüberliegenden Längsseite 11b des Korpus 10 sind mehrere, hier beispielsweise 5 Ausgänge A1, A2, A3, A4, A5 von Ausgangsbuchsen 15a, 15b, 15c, 15d und 15e ausgebildet. Dieser in der Figur 2 dargestellte Anschlußkörper verfügt demnach über einen einzigen Eingang und mehrere Ausgänge.

Wiederum ist an jeden dieser Ausgänge A1 bis A5 je eine Radiofrequenznadel 2 in der bereits beschriebenen Weise mit der jeweiligen Anschlußbuchse 27 anschließbar und die elektrische Spannungsquelle wird an die Eingangsbuchse 14 zur Ausbildung des Eingangs E1 beispielsweise mittels Krokodilklemme oder eines ähnlichen geeigneten Anschlußmittels angeschlossen. Ferner ist auf der Oberseite des Korpus 10 ein Drehschalter 16 ausgebildet, der über eine der Anzahl Ausgänge, hier 5 Ausgänge entsprechende Anzahl Schaltstellungen verfügt, die auf einer Skala 17 mit Ziffern 1 bis 5 gekennzeichnet sind. In jeder dieser Schaltstellungen 1 bis 5 wird der Eingang E1 elektrisch mit einem entsprechenden Ausgang A1 bis A5 des Anschlußkörpers elektrisch verbunden. Ferner weist der Drehschalter 16 noch eine weitere mit Null gekennzeichnete Schaltstellung auf, in der keiner der Ausgänge A1 bis A5 mit dem Eingang E1 elektrisch verbunden ist. Somit ist es möglich, nicht nur die in den Körper des Patienten eingesteckten Radiofrequenznadeln 2 mit ihren Anschlußleitungen 26 und Anschlußbuchsen 17 an jeweils einem Ausgang A1, A2, A3, A4, A5 des Anschlußkörpers 1 anzuschließen, sondern auch die elektrische Spannungsquelle wird direkt an den einzigen Eingang E1 angeschlossen. In der Ausgangsstellung befindet sich hierzu der Drehschalter 16 in Schaltstellung 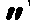 0", bei der keiner der Ausgänge A1 bis A5 mit dem Eingang E1 elektrisch verbunden ist. Sodann kann durch aufeinanderfolgendes Aufsuchen aller übrigen Schaltpositionen 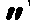 1" bis 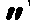 5" jeweils ein entsprechender Ausgang A1 bis A5 elektrisch mit dem Eingang E1 verbunden werden und somit die entsprechende an den jeweiligen Ausgang A1 bis A5 angeschlossene Radiofrequenznadel 2 mit der an den Eingang E1 angeschlossenen elektrischen Spannungsquelle verbunden werden, um die Facettenblockade zu bewirken. Nachdem sämtliche Ausgänge A1 bis A5 durch Aufsuchen der entsprechenden Schaltstellungen mit der elektrischen Spannungsquelle verbunden waren, wird der Drehschalter 16 wieder in Schaltstellung 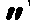 0" bewegt, wodurch sämtliche Ausgänge A1 bis A5 vom Eingang E1 getrennt sind und die Behandlung abgeschlossen ist.

Es ist offensichtlich, daß bei dieser Ausführungsform gemäß Figur 2 eine nochmals erleichterte Handhabung gegenüber dem Ausführungsbeispiel gemäß Figur 1 erzielt wird, da sämtliche Anschlüsse ausgangsseitig wie auch eingangsseitig vor Beginn der Behandlung vorgenommen werden können und lediglich noch der Drehschalter 16 in entsprechenden Zeitabständen betätigt werden muß, um eine Facettenblockade oder auch eine Nervenstimulation zu bewirken.

In der Figur 3 ist eine weitere Ausführungsform des erfindungsgemäßen Anschlußkörpers 1 dargestellt, der sich von dem vorangehend erläuterten Anschlußkörper 1 gemäß Figur 2 lediglich in der Ausbildung des Drehschalters 16 und dessen Schaltstellungen unterscheidet. Wiederum ist ein einziger Eingang E1 und eine Mehrzahl von Ausgängen A1 bis A5 vorgesehen. Der Drehschalter 16 weist hierbei nicht nur 5 Schaltstellungen 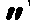 1" bis 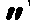 5" auf, in denen jeweils der entsprechende Ausgang A1 bis A5 elektrisch mit dem Eingang E1 verbunden wird, sondern zwischen jeder aufeinanderfolgenden und einen Ausgang mit dem Eingang verbindenden Schaltstellung, beispielsweise zwischen der den Ausgang A1 mit dem Eingang E1 verbindenden Schaltstellung 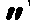 1" und der den Ausgang A2 mit dem Eingang E1 verbindenden Schaltstellung 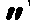 2", jeweils eine weitere Schaltstellung 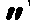 0" vorgesehen ist, in welcher keiner der Ausgänge A1 bis A5 mit dem Eingang E1 verbunden ist. Hierdurch ist es möglich, nach jedem Aufsuchen einer Schaltposition, die einen der Ausgänge A1 bis A5 mit dem Eingang E1 verbindet, durch Drehen des Drehschalters 16 in die nächste Schaltstufe eine Unterbrechung der Verbindung des Eingangs E1 mit einem der Ausgänge A1 bis A5 einzulegen, so daß gefahrlos die richtige Position aller Radiofrequenznadeln 2 geprüft werden kann oder auch andere Maßnahmen am Patienten vorgenommen werden können.

Beiden vorgenannten Ausführungsbeispielen gemäß Figur 2 und Figur 3 ist zueigen, daß lediglich ein Aufsuchen aller Schaltpositionen des Drehschalters, z. B. durch die Drehung im Uhrzeigersinn bewirkt werden muß, um alle ausgangsseitig an die Ausgänge A1 bis A5 angeschlossenen Radiofrequenznadeln einmal mit der eingangsseitig an den Eingang E1 angeschlossenen elektrischen Spannungsquelle zu verbinden. Das Ausbilden einer Facettenblockade wird hierdurch enorm vereinfacht.

Selbstverständlich sind die vorangehend erläuterten Ausführungsbeispiele und insbesondere die Anordnung und Anzahl der Eingänge und Ausgänge lediglich exemplarisch, sie sind im konkreten Anwendungsfall vom Fachmann an die Anzahl zu erwartender Radiofrequenznadeln 2 anzupassen. Somit können auch mehr oder weniger Ausgänge als die hier dargestellten 4 bzw. 5 Ausgänge am Anschlußkörper 1 und auch entsprechend ein oder mehrere Eingänge je nach Ausführungsform und Anzahl der Ausgänge vorgesehen sein. Bei der Ausführungsform gemäß Figur 1 ist stets die gleiche Anzahl Eingänge wie Ausgänge vorhanden und bei den Ausführungsformen gemäß Figuren 2 und 3 ist ein Eingang und eine ausreichende Anzahl Ausgänge mit entsprechender Anzahl Schaltstellungen am Drehschalter 16 vorgesehen.

## Patentansprüche

1. Anschlußkörper zum Anschließen einer Vielzahl von elektrisch leitfähigen Radiofrequenznadeln an eine elektrische Spannungsquelle zur Nervenstimulation und/oder Thermokoagulation eines Patienten, umfassend einen Korpus mit mindestens einem Eingang, an den die elektrische Spannungsquelle anschließbar ist und eine Vielzahl von mit dem mindestens einem Eingang elektrisch verbindbaren Ausgängen, an die jeweils eine Radiofrequenznadel anschließbar ist.

2. Anschlußkörper nach Anspruch 1, **dadurch gekennzeichnet**, daß der Korpus (10) eine Vielzahl von Durchgangsbohrungen (12a, 12b, 12c, 12d) aufweist, in die jeweils ein elektrisch leitfähiger Kontaktstift (13a, 13b, 13c, 13d) eingesetzt ist, so daß an einem Ende jedes Kontaktstiftes ein Eingang für den Anschluß der elektrischen Spannungsquelle und am gegenüberliegenden Ende ein Ausgang für den Anschluß je einer Radiofrequenznadel (2) ausbildbar ist.

3. Anschlußkörper nach Anspruch 2, **dadurch gekennzeichnet**, daß die Kontaktstifte eine solche Länge aufweisen, daß sie mit ihren beiden Enden aus dem Korpus (10) hervorragen.

4. Anschlußkörper nach Anspruch 1, **dadurch gekennzeichnet**, daß eine einen Eingang ausbildende Eingangsbuchse (14) für den Anschluß der elektrischen Spannungsquelle und eine Vielzahl von Ausgängen ausbildende Ausgangsbuchsen (15a, 15b, 15c, 15d, 15e) für den Anschluß jeweils einer Radiofrequenznadel (2) vorgesehen sind und der Anschlußkörper (1) einen Schalter (16) aufweist, mittels dessen je eine Ausgangsbuchse mit der Eingangsbuchse des Anschlußkörpers (1) elektrisch verbindbar ist.

5. Anschlußkörper nach Anspruch 4, **dadurch gekennzeichnet**, daß der Schalter der Anzahl Ausgangsbuchsen entsprechende Schaltstellungen für die elektrische Verbindung je einer Ausgangsbuchse mit der Eingangsbuchse und eine weitere Schaltstellung aufweist, in welcher keine der Ausgangsbuchsen mit der Eingangsbuchse verbunden ist.

6. Anschlußkörper nach Anspruch 4, **dadurch gekennzeichnet**, daß der Schalter der Anzahl Ausgangsbuchsen entsprechende Schaltstellungen für die elektrische Verbindung je einer Ausgangsbuchse mit der Eingangsbuchse aufweist und zwischen zwei benachbarten Schaltstellungen zum Verbinden einer Ausgangsbuchse mit der Eingangsbuchse jeweils eine weitere Schaltstellung vorgesehen ist, in welcher keine Ausgangsbuchse mit der Eingangsbuchse verbunden ist.

7. Anschlußkörper nach einem der Ansprüche 4 bis 6, **dadurch gekennzeichnet**, daß der Schalter als Drehschalter ausgebildet ist.

8. Anschlußkörper nach einem der Ansprüche 1 bis 7, **dadurch gekennzeichnet**, daß der mindestens eine Eingang und die Ausgänge für den Anschluß von Steckbuchsen und/oder Krokodilklemmen vorgesehen sind.

9. Anschlußkörper nach einem der Ansprüche 1 bis 8, **dadurch gekennzeichnet**, daß der Korpus 10 eine quaderförmige Gestalt aufweist und der mindestens eine Eingang auf einer Längsseite (11a) und die Ausgänge auf der gegenüberliegenden Längsseite (11b) des Korpus (10) ausgebildet sind.

10. Anschlußkörper nach einem der Ansprüche 1 bis 9, **dadurch gekennzeichnet**, daß der Korpus aus einem thermoplastischen Kunststoff, wie PVC hergestellt ist.
